# DEMANDE DE BREVET EUROPEEN

(11) **EP 0 865 759 A1**
(43) Date de publication de la demande: **23.09.1998**
(21) Numéro de dépôt: 98400632.0
(22) Date de dépôt: 18.03.1998
(51) Int. Cl.: A61B 1/267, H04N 5/232

(54) **Dispositif de prise de vue à effet stroboscopique**

(30) Priorité: 21.03.1997 FR 9703458
(71) Demandeur: Sopro, 13011 Marseille (FR)
(72) Inventeur: Boyer, Philippe, 13006 Marseille (FR); Mazuir, Alain, 13015 Marseille (FR)
(74) Mandataire: Hasenrader, Hubert

(57) **Abrégé**

L'invention concerne un dispositif de prise de vue (20) à effet stroboscopique destiné à visualiser un phénomène répétitif, notamment un phénomène sensiblement périodique de fréquence instantanée F.

Selon l'invention, ce dispositif comprend une source de lumière continue (24) destinée à éclairer ledit phénomène, un dispositif de déclenchement (28) permettant, à partir du phénomène, de générer des impulsions synchrones audit phénomène, lesdites impulsions étant périodiques et de même fréquence F que le phénomène si ledit phénomène est sensiblement périodique, un dispositif capteur d'image (30), de préférence à transfert de charge (CCD), présentant un temps d'exposition tₑ, et des moyens de commande (32) du dispositif capteur d'image, reliés audit dispositif de déclenchement (28) de façon à pouvoir commander de façon régulière le début de l'exposition (tₑ) du dispositif capteur d'image (30) en réponse à au moins l'une des impulsions du dispositif de déclenchement (28).

Application : visualisation de cordes vocales (22) en mouvement.

## Description

L'invention conceme un dispositif de prise de vue à effet stroboscopique destiné à visualiser un phénomène répétitif, notamment un phénomène sensiblement périodique de fréquence instantanée F.

De façon plus précise, l'invention concerne une installation de prise d'image qui permet de suivre le mouvement des cordes vocales d'un patient, qui soit d'un emploi aisé pour le médecin spécialisé en phoniatrie et qui présente un coût moindre que les installations disponibles actuellement.

A l'heure actuelle, pour des examens portant sur l'analyse des cordes vocales, les phoniatres utilisent une caméra classique filmant en continu couplée à une source de lumière stroboscopique. Le dispositif d'examen des cordes vocales (endoscope, laryngoscope, fibroscope ou nasofibroscope) comprend à la fois la caméra et la source de lumière nécessaire à l'éclairage des cordes vocales.

Les cordes vocales vibrant à une fréquence comprise entre quelques Hz et quelques KHz, on souhaite les filmer de façon qu'elles apparaissent fixes à l'écran ou bien avec des mouvements très ralentis (effet stroboscopique) afin que le médecin puisse suivre le parcours des cordes vocales et établir son diagnostic. Pour cela, un microphone est posé à proximité du larynx du patient et une caméra filme en continu le mouvement des cordes vocales. L'effet stroboscopique est obtenu grâce à une source de lumière stroboscopique qui est une source de lumière qui éclaire les cordes vocales avec des éclairs de lumière dont la fréquence est en rapport avec celle des vibrations des cordes vocales, un seul éclair de lumière étant émis par période du signal de trame de la caméra.

Les examens de phoniatrie requièrent deux lampes distinctes : l'une de type halogène traditionnel, fournissant une lumière continue et utilisée pendant l'examen général du site O.R.L, et une autre de type xénon, foumissant une lumière discontinue froide (flash de forte puissance) et agissant donc comme un stroboscope utilisé seulement pendant l'analyse vibratoire des cordes vocales. Il est à noter que ces deux lampes éclairent le site O.R.L. avec des lumières de température de couleur différentes, de sorte qu'elles produisent des images différentes des cordes vocales, ce qui est une source de problèmes pour le diagnostic du phoniatre.

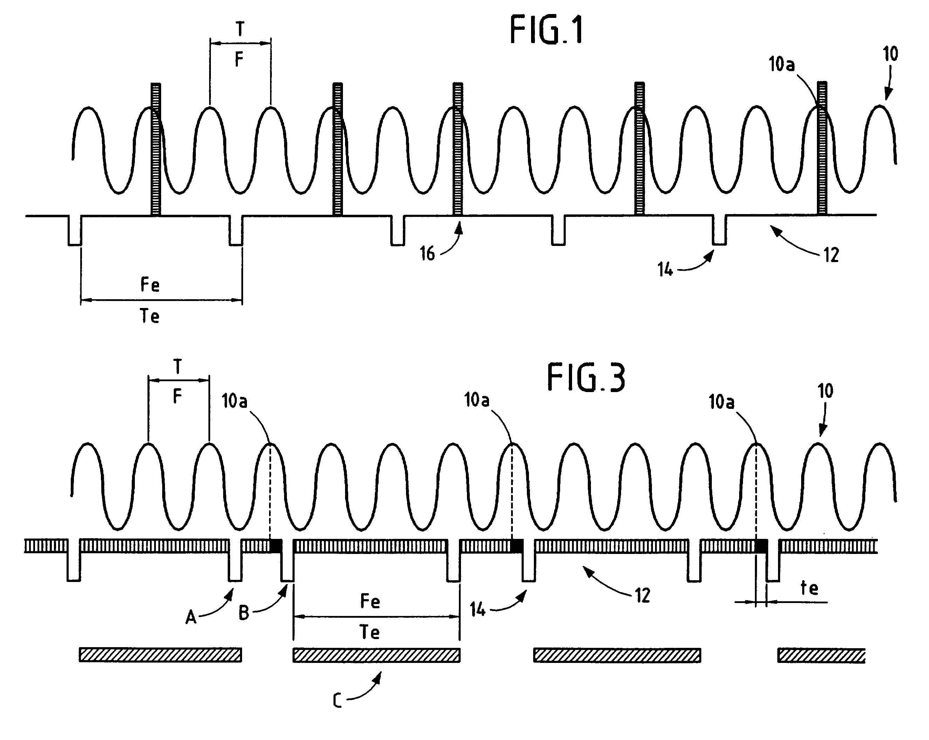

On se reportera à la figure 1 qui illustre le fonctionnement du dispositif de prise de vue de l'art antérieur grâce à un diagramme temps. Le micro génère des oscillations électriques composées d'une série de courbes de type sinusoïdal superposées dont on extrait l'une d'entre elles (la courbe de fréquence fondamentale Fo ou une courbe de fréquence multiple de Fo), cette courbe 10 de forme sensiblement sinusoïdale est périodique et de fréquence instantanée F. Un dispositif de déclenchement de type trigger analyse la courbe sinusoïdale 10 en sélectionnant l'avènement d'un niveau de signal présélectionné au cours de l'oscillation électrique 10, par exemple le trigger est réglé pour repérer chaque sommet 10a de la sinusoïde. De préférence, le capteur d'image utilisé est une caméra de type CCD dont le signal de trame, périodique, est représenté par la courbe 12, le signal de trame périodique présentant une fréquence Fₑ ou une période Tₑ. Le signal de trame 12 de la caméra CCD présente une forme en créneau avec, pour chaque début de période (ou créneau), ce que l'on appelle un top trame (référence 14 sur la figure 1).

Le trigger est relié à une source de lumière stroboscopique qui éclaire les cordes vocales par un éclair 16 ou flash de forte puissance une fois par période du signal de trame 12 de la caméra CCD, cet éclair 16 étant en phase avec la fréquence F des oscillations électriques 10, c'est-à-dire, dans l'exemple illustré à la figure 1, au sommet de la sinusoïde.

A titre d'exemple illustratif, sur les figures 1 et 3, on a choisi de représenter une courbe sinusoïdale 10 de fréquence F = 133 Hz (T = 7,5 ms) et le signal de trame 12 représenté possède une fréquence Fₑ = 50 Hz (Tₑ = 20 ms).

Ainsi, dans l'art antérieur, les cordes vocales sont éclairées par la source stroboscopique pendant une durée de quelques millisecondes, à une fréquence variable (apériodique) proche de la fréquence Fₑ de la caméra CCD.

Les explications qui précèdent sont valables pour réaliser la visualisation des cordes vocales dans une position fixe sur l'écran relié à la caméra CCD. Si l'on désire un effet de ralenti du mouvement des cordes vocales, il faut alors décaler légèrement la phase des éclairs : le premier éclair sera par exemple au sommet de la sinusoïde, le second éclair au sommet de la sinusoïde + T0 (T0 est un temps très petit par rapport à 1/F), puis un troisième éclair au sommet de la sinusoïde +2T0, etc.

On comprend que le dispositif de l'art antérieur requiert l'utilisation d'une source de lumière stroboscopique particulière envoyant des flashs de forte puissance et qui, de plus, permet de générer une image fixe ou en mouvement des cordes vocales différente de l'image directe observée par le médecin avec une source de lumière continue.

L'invention vise à fournir un dispositif de prise de vue à effet stroboscopique qui ne présente pas les inconvénients de l'art antérieur, notamment, mais non exclusivement, dans le cas d'une installation phoniatrique pour l'examen des cordes vocales.

Pour atteindre ce but, selon l'invention, le dispositif de prise de vue se caractérise en ce qu'il comprend :
- une source de lumière continue destinée à éclairer ledit phénomène,
- un dispositif de déclenchement permettant, à partir du phénomène, de générer des impulsions synchrones audit phénomène, lesdites impulsions étant périodiques et de même fréquence F que le phénomène si ledit phénomène est sensiblement périodique,
- un dispositif capteur d'image présentant un temps d'exposition tₑ, et un signal de trame de période Tₑ,
- des moyens de commande du dispositif capteur d'image, reliés audit dispositif de déclenchement de façon à pouvoir commander de façon régulière le début de l'exposition (tₑ) du dispositif capteur d'image en réponse à au moins l'une des impulsions du dispositif de déclenchement,
- des moyens de traitement de l'image permettant de transformer le signal généré par ledit dispositif capteur d'image en un signal vidéo, et
- des moyens de stockage du signal vidéo émis par les moyens de traitement de l'image,
- lesdits moyens de commande commandant en outre la remise à zéro du signal de trame du dispositif capteur d'image et l'activation desdits moyens de stockage en réponse à la fin du temps d'exposition (tₑ) du dispositif capteur d'image.

On comprend que, grâce au moyen de commande du dispositif capteur d'image, l'effet stroboscopique est assuré par le dispositif capteur d'image lui-même et non par la source de lumière qui est maintenant une source standard éclairant en continu, de moindre coût que le générateur de lumière de l'art antérieur. Cette source de lumière est donc utilisable pour d'autres usages que l'analyse vibratoire des cordes vocales puisque c'est également une source de lumière de type standard qui est utilisée en endoscopie classique pour l'examen général du site O.R.L.

Selon l'invention, le dispositif capteur d'image ne filme pas en continu mais le début de l'exposition du dispositif capteur d'image est synchronisé avec les vibrations des cordes vocales.

Selon un mode préféré de mise en oeuvre, ledit dispositif de prise de vue se caractérise en ce qu'il comporte, en outre, des moyens de lecture du signal vidéo contenu dans lesdits moyens de stockage, les moyens de lecture étant indépendants desdits moyens de stockage et pouvant lire en boucle ledit signal vidéo.

D'autres caractéristiques et avantages de la présente invention apparaîtront mieux à la lecture de la description qui suit d'un mode de mise en oeuvre de l'invention donné à titre d'exemple non limitatif. La description se réfère aux figures annexées sur lesquelles :
- la figure 1, déjà décrite, est un diagramme temps représentant le principe de fonctionnement du dispositif de prise de vue à effet stroboscopique de l'art antérieur ;
- la figure 2 représente de façon schématique le diagramme synoptique de l'installation de prise de vue selon l'invention ; et
- la figure 3 est un diagramme temps illustrant le principe de fonctionnement du dispositif de prise de vue selon l'invention.

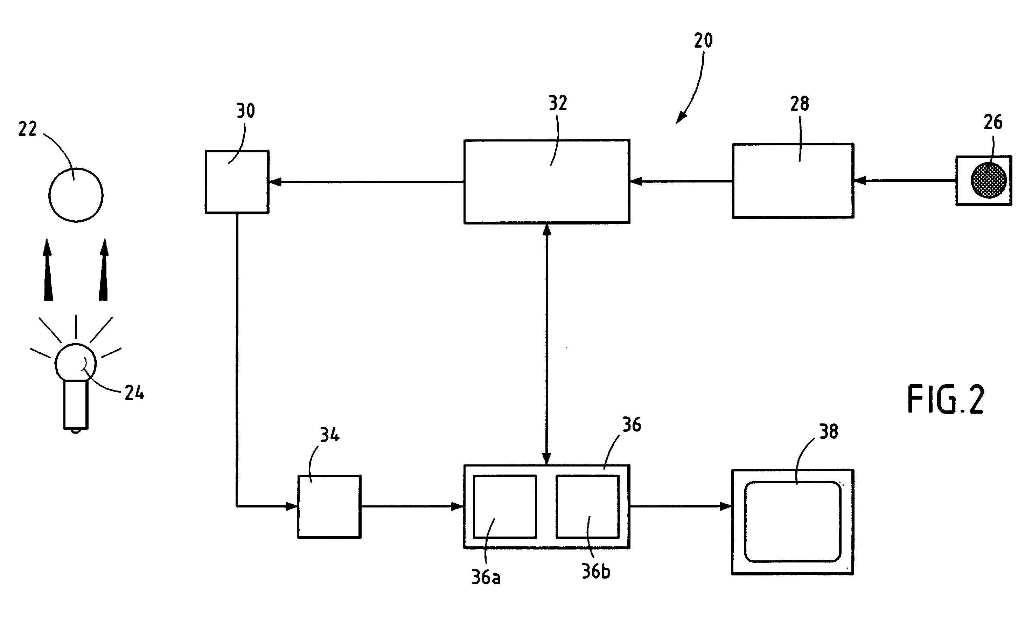

On se reportera à la figure 2 représentant de façon schématique le dispositif de prise de vue 20 de la présente invention pour la visualisation de cordes vocales 22 en mouvement qui constituent le phénomène que l'utilisateur du dispositif selon l'invention souhaite analyser.

Une source ou générateur de lumière continue 24 éclaire en permanence les cordes vocales 22 en mouvement. Un microphone 26 placé à proximité des cordes vocales permet de transformer les vibrations sonores du phénomène étudié, c'est-à-dire le mouvement des cordes vocales, en oscillations électriques 10 dont la courbe représentative, de forme sensiblement sinusoïdale, est visible sur les figures 1 et 3. Des moyens de traitement du signal 28 sont reliés au microphone 26 et font partie d'un dispositif de déclenchement de type trigger permettant, comme dans le dispositif de l'art antérieur, d'analyser le signal sensiblement sinusoïdal afin d'en repérer les maximums correspondant au sommet 10a de cette courbe sinusoïdale 10.

On comprend que les moyens de traitement du signal 28 permettent d'analyser les oscillations électriques issues du microphone 26 pour générer une impulsion à chaque période du signal de type sinusoïdal engendré par les oscillations électriques, les impulsions ainsi générées étant, de préférence, en phase avec le phénomène à observer, c'est-à-dire le mouvement des cordes vocales ou encore le signal sinusoïdal 10 représentatif de ce mouvement.

Un dispositif capteur d'image 30, de préférence du type à transfert de charge ou CCD, normalement en position d'obturation, est placé face aux cordes vocales 22 en mouvement.

Les moyens de commande du dispositif de prise de vue 20 selon la présente invention comportent un générateur d'horloge 32 relié, d'une part, aux moyens de traitement du signal 28 et, d'autre part, au capteur d'image CCD 30 de façon à constituer une interface entre ces deux éléments.

Le dispositif de prise de vue 20 comporte en outre des moyens de traitement de l'image 34 constitués par un module de traitement de l'image 34 permettant de transformer le signal généré par le dispositif capteur d'image CCD en un signal vidéo. Le générateur d'horloge 32 et le module de traitement de l'image 34 sont reliés à un module mémoire d'image 36 présentant un circuit d'écriture 36a et un circuit de lecture 36b indépendants l'un de l'autre.

Le circuit d'écriture 36a constitue des moyens de stockage du signal vidéo émis par le module de traitement de l'image 34 et le circuit de lecture 36b du module mémoire d'image 36, associé à un moniteur 38 constituant un écran de visualisation, constitue des moyens de lecture de l'information, c'est-à-dire de l'image générée par le capteur d'image CCD pendant le temps d'exposition de ce capteur CCD 30.

Une analyse séquentielle du fonctionnement du dispositif de prise de vue selon l'invention va maintenant être présentée en relation avec la figure 3. Sur cette figure 3, on retrouve la courbe sinusoïdale 10 représentative des oscillations électriques issues des cordes vocales 22 en mouvement, ce signal 10 présentant une fréquence F pouvant varier au cours du temps mais qui est constante sur un intervalle de temps court comme celui qui est représenté sur la figure 3, de sorte que l'on désignera cette fréquence F sous le terme de fréquence instantanée. La courbe du bas de la figure 3 illustre le signal de trame 12 du capteur CCD, le début de chaque période Tₑ du signal de trame 12 étant repéré par un signal impulsionnel 14 appelé top trame, l'obturation du capteur CCD étant représentée par les zones avec des hachures droites et l'exposition du capteur CCD étant représentée par les zones noires de faible durée (quelques millisecondes).

On se placera au top trame A qui débute une nouvelle période du signal de trame du capteur d'image CCD 30, le capteur d'image CCD 30 étant obturé au début de la période du signal de trame du capteur CCD 30 qui suit le moment A. Le générateur d'horloge 32 commande l'exposition (zones noires) du capteur CCD 30 lorsque le générateur d'horloge 32 reçoit, à partir du module traitement du signal 28, l'information ou impulsion signalant un sommet 10a de la courbe sinusoïdale 10.

A la fin du temps d'exposition pendant lequel le capteur d'image 30 ou détecteur d'image CCD a fait l'acquisition de l'image résultant de la position des cordes vocales 22 à ce moment-là (voir B sur la figure 3), le générateur d'horloge 32 génère un top trame forcé en direction du capteur d'image 30, ce top trame forcé B constituant une remise à zéro intempestive du capteur CCD en réponse à la fin du temps d'exposition qui correspond ainsi, de façon classique, à la fin du signal de trame du capteur CCD 30.

Après le top trame intempestif (point B), le générateur d'horloge 32 a commandé à nouveau l'obturation du capteur CCD 30 et il déclenche également une période d'écriture en mémoire dans le circuit d'écriture 36a de la mémoire d'image 36, des informations vidéo générées par le module de traitement vidéo 34 à partir des informations recueillies par le capteur CCD 30 pendant le temps d'exposition précédant le point B.

De préférence, la période d'écriture en mémoire C, représentée sur la figure 3 par une zone hachurée en trait oblique, dure une période entière Tₑ du signal de trame 12 du capteur CCD, le capteur CCD étant toujours obturé pendant cette période d'écriture en mémoire C.

A la fin de la période d'écriture en mémoire C, on se retrouve dans la même situation qu'au point A, c'est-à-dire au début d'une nouvelle période du signal de trame : après repérage d'un nouveau sommet 10a de la sinusoïde 10, le générateur d'horloge 32 commande successivement l'exposition du capteur CCD 30, le retour top trame intempestif (point B) du signal de trame du capteur CCD 30 puis la période d'écriture en mémoire (C) dans le circuit d'écriture 36a de la mémoire d'image 36.

La visualisation sur le moniteur 38 de l'image obtenue est permise grâce au fait que le circuit de lecture 36b lit en boucle les informations contenues dans le circuit d'écriture 36a et génère une image vidéo périodique de fréquence f, 50 Hz par exemple, correspondant à la fréquence f de fonctionnement de l'écran de visualisation ou moniteur 38.

On souhaite que le dispositif capteur d'image CCD 30 soit exposé régulièrement et aussi souvent que possible jusqu'à concurrence d'une fois par période Tₑ du signal de trame. Pour cela, de préférence, le dispositif de prise de vue selon l'invention est apte à commander le début de l'exposition tₑ du dispositif capteur d'image 30 au moins une fois toutes les n périodes Tₑ du signal de trame, avec n > 2.

De façon plus précise, le dispositif de prise de vue décrit est apte à commander le début de l'exposition tₑ du dispositif capteur d'image 30 au moins une fois toutes les trois périodes Tₑ du signal de trame (de préférence au moins une fois toutes les deux périodes Tₑ) et au plus une fois par période Tₑ du signal de trame.

On comprend que c'est pendant la période d'écriture en mémoire C, des informations recueillies par le capteur CCD 30 dans le circuit d'écriture 36a, que l'image disponible lisible par le circuit de lecture 36b est "rafraîchie".

Ainsi, selon la présente invention, le générateur d'horloge ne foumit pas un signal périodique et le signal de trame 12 du capteur d'image 30 est également apériodique puisqu'il alterne des périodes d'écriture en mémoire, d'une durée égale à la période Tₑ du signal de trame 12, et des périodes d'attente d'un nouveau maximum 10a du signal sinusoïdal 10 représentatif du mouvement des cordes vocales, déclenchant le temps d'exposition te du capteur d'image CCD à la fin duquel le générateur d'horloge 32 génère un top trame 14 intempestif (B). On comprend que le temps séparant les points A et B varie à chaque fois mais que cet écart est d'autant plus court que la fréquence F de vibration des cordes vocales 22 est élevée.

On comprend que, grâce à l'invention, en utilisant des circuits électroniques adaptés, on réalise une caméra de type CCD à effet stroboscopique dans laquelle c'est l'exposition du capteur CCD 30, déclenchée en réponse à un événement extérieur, qui réalise l'effet stroboscopique, les cordes vocales étant éclairées en continu par un générateur de lumière standard.

Dans la description précédente, on a envisagé le cas de la visualisation des cordes vocales en position fixe mais, comme dans le cas de l'art antérieur, on peut filmer le mouvement ralenti des cordes vocales, en décalant légèrement le début du temps d'exposition du capteur CCD 30 par rapport au sommet 10a de la sinusoïde 10.

On comprend également que la description qui vient d'être faite reste valable en tout point si, au lieu de repérer le sommet 10a de la sinusoïde 10, le module de traitement du signal 28 repère une autre position du signal sinusoïdal 10, en générant une impulsion à chaque itération de cette position.

De façon analogue, le dispositif de prise de vue objet de la présente invention permet de visualiser tout phénomène répétitif, même apériodique, le nodule de traitement du signal 28 étant alors apte à repérer l'avènement dudit phénomène pour générer à ce moment une impulsion entraînant la commande de l'exposition du capteur CCD 30.

On comprend que la description qui vient d'être faite est liée à l'utilisation d'un capteur de type CCD 30 classique pour lequel le temps d'exposition est placé à la fin du signal de trame mais, sans sortir du cadre de l'invention, on peut utiliser d'autres types de capteur CCD ou toute autre caméra commandée et effectuant sa prise d'image de façon électronique.

De façon particulièrement avantageuse, le dispositif de prise de vue selon la présente invention fonctionne « en mode normal », c'est-à-dire qu'il filme en continu, en l'absence d'un signal de déclenchement, c'est-à-dire, en l'absence d'impulsions synchrones audit phénomène répétitif (par exemple les sommets 10a périodiques de la sinusoïde). En effet, dans ce cas, il n'y a pas de top trame forcé (voir B sur la figure 3) et le capteur d'image CCD 30 continue à être exposé.

## Revendications

1. Dispositif de prise de vue (20) à effet stroboscopique destiné à visualiser un phénomène répétitif, notamment un phénomène sensiblement périodique de fréquence instantanée F, caractérisé en ce qu'il comprend :
une source de lumière continue (24) destinée à éclairer ledit phénomène,
un dispositif de déclenchement (28) permettant, à partir du phénomène, de générer des impulsions synchrones audit phénomène, lesdites impulsions étant périodiques et de même fréquence F que le phénomène si ledit phénomène est sensiblement périodique,
un dispositif capteur d'image (30) présentant un temps d'exposition tₑ, et
des moyens de commande (32) du dispositif capteur d'image, reliés audit dispositif de déclenchement (28) de façon à pouvoir commander de façon régulière le début de l'exposition (tₑ) du dispositif capteur d'image (30) en réponse à au moins l'une des impulsions du dispositif de déclenchement (28).

2. Dispositif de prise de vue (20) selon la revendication 1, caractérisé en ce que le dispositif capteur d'image (30) est un dispositif à transfert de charge (CCD).

3. Dispositif de prise de vue selon la revendication 1 ou 2, caractérisé en ce que ledit capteur d'image (30) possède un signal de trame de période Tₑ et en ce qu'il comporte en outre :
des moyens de traitement de l'image (34) permettant de transformer le signal généré par ledit dispositif capteur d'image (30) en un signal vidéo,
des moyens de stockage (36a) du signal vidéo émis par les moyens de traitement de l'image (34),
des moyens de lecture (36b) du signal vidéo contenu dans lesdits moyens de stockage (36a), et
en ce que lesdits moyens de commande (32) peuvent en outre commander la remise à zéro du signal de trame du dispositif capteur d'image (30) et l'activation desdits moyens de stockage (36a) en réponse à la fin du temps d'exposition (tₑ) du dispositif capteur d'image (30).

4. Dispositif de prise de vue selon l'une des revendications précédentes, caractérisé en ce qu'il comprend en outre un module mémoire d'image (36) présentant un circuit d'écriture (36a) et un circuit de lecture (36b), en ce que lesdits moyens de stockage comprennent ledit circuit d'écriture (36a) dudit module mémoire d'image et en ce que lesdits moyens de lecture comprennent ledit circuit de lecture (36b) dudit module mémoire d'image et un écran de visualisation (38).

5. Dispositif de prise de vue selon la revendication 3, caractérisé en ce qu'il est apte à commander le début de l'exposition (tₑ) dudit dispositif capteur d'image (30) au moins une fois toutes les n périodes Tₑ dudit signal de trame, avec n ≥ 2.

6. Dispositif de prise de vue selon la revendication 5, caractérisé en ce qu'il est apte à commander le début de l'exposition (tₑ) dudit dispositif capteur d'image (30) au moins une fois toutes les trois périodes Tₑ dudit signal de trame et au plus une fois par période Tₑ dudit signal de trame.

7. Dispositif de prise de vue selon l'une des revendications précédentes, caractérisé en ce qu'il comprend en outre :
un microphone (26) destiné à transformer les vibrations sonores du phénomène en oscillations électriques (10), et
en ce que ledit dispositif de déclenchement comporte des moyens de traitement du signal (28) reliés audit microphone et permettant d'analyser lesdites oscillations électriques (10) pour générer une impulsion à chaque période desdites oscillations électriques (10).

8. Utilisation du dispositif de prise de vue selon l'une des revendications précédentes à la visualisation de cordes vocales (22) en mouvement.
